# EUROPEAN PATENT SPECIFICATION

(11) **EP 2 196 206 B1**
(45) Date of publication and mention of the grant of the patent: **17.07.2019**
(21) Application number: 08835121.8
(22) Date of filing: 03.10.2008
(51) Int. Cl.: A61K 31/485, A61K 8/49, A61P 17/00, A61Q 19/00, C07D 489/02

(54) **REMEDY FOR RELIEVING SKIN TROUBLES COMPRISING MORPHINAN DERIVATIVE OR PHARMACOLOGICALLY ACCEPTABLE ACID ADDITION SALT THEREOF AS THE ACTIVE INGREDIENT**
HEILMITTEL ZUR LINDERUNG VON HAUTPROBLEMEN MIT EINEM MORPHINANDERIVAT ODER PHARMAKOLOGISCH VERTRÄGLICHEN SÄURADDITIONSSALZ DARAUS ALS WIRKSTOFF
REMÈDE DESTINÉ À SOULAGER DES TROUBLES CUTANÉS COMPRENANT UN DÉRIVÉ DE MORPHINANE OU L'UN DE SES SELS D'ADDITION D'ACIDE ACCEPTABLES SUR LE PLAN PHARMACOLOGIQUE EN TANT QUE PRINCIPE ACTIF

(30) Priority: 05.10.2007 JP 2007262015
(43) Date of publication of application: 16.06.2010
(73) Proprietor: Toray Industries, Inc., Tokyo, 103-8666 (JP); Torii Pharmaceutical Co., Ltd., Chuo-ku Tokyo 103-8439 (JP)
(72) Inventor: ONA, Shinji, Tokyo 103-8666 (JP); TAKESHITA, Koichiro, Tokyo 103-8666 (JP); ANDO, Naoki, Tokyo 103-8666 (JP); YAMASHITA, Masahiro, Tokyo 103-8666 (JP)
(74) Representative: Kador & Partner PartG mbB
(86) International application number: PCT/JP2008/068092
(87) International publication number: WO 2009/044883

(56) References cited:
- EP-A1- 0 897 726
- WO-A1-98/23290
- WO-A1-99/05146
- WO-A1-2004/074277
- WO-A1-2006/095836
- WO-A2-2007/053194
- JP-A- 3 204 815
- JP-A- 2005 220 027

## Description

### Technical Field

The present invention relates to the provision of a novel medicine comprising a morphinan derivative or any of its pharmacologically permissible acid addition salts useful for therapy to improve skin properties via a skin moisture retaining effect.

### Background Art

It is known that skin roughening, skin drying, skin darkening, are caused by the functional decline of the skin barrier, and for the purpose of improving skin properties, toilet water, foundations, emulsions, horse oil, ointments, have been being used. As skin property-improving medicines, medicines for external application including heparinoids such as Hirudoid, Airleet, Kuradoid, Seleloiz and Besoften, urea, hyaluronic acid and collagen are formulated for treating such indications as asteatosis cutis and keratodermia tylodes palmaris progressiva. Further, it is also reported that cosmetics comprising, as a main ingredient, an extract obtained by extracting the roots, subterranean stems or leaves of *Phyllostachys bambusoides, Phyllostachys nigra* or *Phyllostachys heterocycle* using a hydrophilic organic solvent (Patent Literature 1) and cosmetics comprising, as a main ingredient, an extract of bamboo stems or branches (Patent Literature 2) are effective for improving the dry skin accompanied by astetic change. It is also reported that some plant extracts promote the liberation of β-endorphins from keratinocytes, for promoting the moisture retention of the skin (Non-Patent Literature 7).

In general, in the healthy horny layer of the skin, the stratum corneum intercellular lipids form a double lipid layer for retaining water, and since the low molecular water soluble substances in the keratin cells keep flexibility, the sebum inhibits percutaneous water transpiration, to keep the skin surface lubricating. Ceramides play important roles of retaining skin water and maintaining the barrier function of the horny layer, and in an alkaline region, it is considered that ceramidase activity rises to promote the hydrolysis reactions from ceramides into fatty acids and sphingosine, resulting in dry skin (Non-Patent Literature 1) . About 60% of the patients treated with hemodialysis are found to suffer from itching (Non-Patent Literatures 2 and 3), and on the other hand, about 90% of them are found to experience dry skin and to record significant decline in the water content of the horny layer and significant rise in the pH value of the skin (Non-Patent Literature 4). Further, in addition to these, also in the xeroderma most frequently observed with the patients treated with hemodialysis, the amount of skin surface lipids is found to decrease (Non-Patent Literature 5), and it is also reported that these patients are higher than healthy persons in the average pH value of the skin and can have itching reduced if they are coated with an acid cream having a lactic acid buffer as an aqueous phase (Non-Patent Literature 6). Skin drying is often accompanied by aging, atopic dermatitis and the xeroderma occurring especially in winter, and there is a demand for the development of a moisture retaining agent that has both the water retaining function and the barrier function.

The morphinan compound used as an active ingredient in this invention displays opioid κ agonism and the applications of the morphinan compound based on its analgesic activity, diuretic activity and antitussive activity are already disclosed (Patent Literature 3). Further, already disclosed are applications as brain cell protective (Patent Literature 4), antipruritic agent (Patent Literature 5), hyponatremia remedy (Patent Literature 6), ORL-1 receptor antagonist (Patent Literature 7), remedy for neuropathic pain (Patent Literature 8), remedy for psychoneurosis (Patent Literature 9), remedy for drug addiction (Patent Literature 10), remedy for sepsis (Patent Literature 11), antipruritic agent for pruritus caused by multiple sclerosis (Patent Literature 12. The Patent Literature 5 discloses the antipruritic effect by κ agonist via the central function.

With regard to other opioid-based drugs such as morphine than those described above, the drugs, the skin improving effects of which are disclosed, include the following. It is reported that opioids for external application are effective for sebaceous gland disorders such as acne and burns (Patent Literature 13) and that β-endorphine are capable of increasing the water content of the skin, improving the barrier function of the skin, promoting the turnover, and preventing the initial progression of aging (Non-Patent Literature 7). It was found in 1999 that β-endorphins exist in the skin, and it is reported that *in vitro,* the β-endorphins (1) increase natural moisturizing factors (amino acids, filaggrin), (2) increase a turnover regulatory factor (cytokeratin 1), (3) increase a cell adhesion factor (desmosome), (4) increase the envelope of the horny layer (involucrin, loricrin), and (5) increase an anchoring complex (laminin 5). With regard to the effects of opioids on skin cells, it is reported that β-endorphins as an agonist enhance the production of cytokeratin 16 as a differentiation marker of epidermis cornification cells (Non-Patent Literature 8) and that β-endorphins stimulate the wandering of keratinocytes (Non-Patent Literature 9).

It is known that opioids have analgesic action and on the other hand also function as a chemical mediator of itching, and it is reported that endogenous opioid peptides such as β-endorphins and enkephalins cause itching (Non-Patent Literature 10). Though details are not clear, it is generally said that µ-receptors and δ-receptors participate in inhibiting pain and inducing itching and that κ-receptors participate in inhibiting pain and itching (Non-Patent Literature 11). Opioid-based drugs reported to have an antipruritic effect include naloxone and naltrexone (Non-Patent Literatures 12 and 13) and it is reported that specific morphinan compounds as active ingredients of the present invention exhibit an antipruritic effect (Non-Patent Literatures 14, 15 and 16). Further, among the drugs having µ-receptor blocking activity, some are reported to be used for therapy such as prevention of chronic pruritus (Patent Literature 14) and there is a prior art document concerning the therapy of itching via the action of inhibiting the peripheral sensory nerves in the skin by selective inhibition of glutamic acid receptors (Patent Literature 15).

The itching of the skin is defined as "a sense of inclining to scratch the skin." The dry state of the skin and the degree of itching generally show a positive correlation, and it is well known that the improvement of the dry state of the skin results in the reduction of itching. On the other hand, there is itching of the skin not accompanied by any abnormal skin property (Non-Patent Literature 17), and the relation between central itching and skin properties is not clear. The central itching caused by excessive reaction with the itching signals generated by the unbalance of intracerebral opioid peptides has nothing to do with abnormal properties of the skin.

Patent Literature 16 discloses the use of kappa-receptor agonists for the treatment of pruritus caused by multiple sclerosis. It further reports that kappa-receptor agonists having 4,5-epoxymorphinan skeleton may be used as anti-pruritic agent. Patent Literature 17 discloses the use of kappa-receptor agonists comprising salts of nalfurafine for the treatment of pruritus in dermatosis.

Non-Patent Literature 18 discloses nalfurafine as a kappa-opioid receptor agonists for the treatment of uremic pruritus and refers to the effect of TRK-820 against itching in patients undergoing hemodialysis and/or having atopic dermatitis.

Also Non-Patent Literature 19 and 20 disclose TRK-820 being effective against itching in patients having renal diseases or undergoing hemodialysis.

Therefore, prior art documents do not disclose the skin property-improving effect of the active ingredient having a specific morphinan skeleton of this invention at all.

[Patent Literature 1] JP5-124930A
[Patent Literature 2] JP7-187990A
[Patent Literature 3] WO93/015081A
[Patent Literature 4] WO95/003307A
[Patent Literature 5] WO98/023290A
[Patent Literature 6] WO99/005146A
[Patent Literature 7] JP2000-53572A
[Patent Literature 8] WO01/014383A
[Patent Literature 9] WO02/078744A
[Patent Literature 10] WO99/011289A
[Patent Literature 11] WO02/089845A
[Patent Literature 12] WO06/095836A
[Patent Literature 13] US Patent No. 5834480
[Patent Literature 14] JP 2004-352714A
[Patent Literature 15] JP2004-107209A
[Patent Literature 16] WO 2006/095836 A1
[Patent Literature 17] EP 0897 726 A1

[Non-Patent Literature 1] "Purification and Biochemical Characterization of Membrane-bound Epidermal Ceramidases from Guinea Pig Skin," (Yukihiro Yada et al.), The Journal of Biological Chemistry, USA, American Society for Biochemistry and Molecular Biology, May 26 1995, Vol. 270, No. 21, pages 12677-12684
[Non-Patent Literature 2] "Itching up date: Effectiveness of κ-opioid Agonists for Itching of Dialysis Patients (in Japanese)," (Hironari Kumagaya et al.), MB Derma, Zen-Nihon Byoin Shuppan-kai (= Publishing Association of All Japanese Hospitals), August 25 2005, Vol. 104, pages 59-64
[Non-Patent Literature 3] "Uraemic Pruritus," (Idit F. Schwartz et al.), Nephrology, Dialysis, Transplantation, official publication of the European Dialysis and Transplant Association - European Renal Association, United Kingdom, Oxford University Press, 1999, Vol, 14, No. 4, pages 834-839
[Non-Patent Literature 4] "Cutaneous Lesions of Dialysis Patients. General Remarks (in Japanese)," (Akira Hattori), Rinsho Toseki (= Clinical Dialysis), Nihon Medical Center, October 1995, Vol. 11, No. 13, pages 1877-1881
[Non-Patent Literature 5] "Substances Relating to Complications Observed with Uremia and Chronic Dialysis X) Skin Disorders (in Japanese)," (Akira Hattori), Rinsho Toseki (= Clinical Dialysis), Nihon Medical Center, August 10 2005, Vol. 21, No. 9, pages 1237-1242
[Non-Patent Literature 6] "Skin Changes in Hemodialysis Patients (in Japanese)," (Akira Hattori et al.), Rinsho Hifuka (= Clinical Dermatology), Igaku-Shoin Ltd., January 1990, Vol. 44, No. 1, pages 21-24
[Non-Patent Literature 7] "New Role of β-endorphin in Skin. (in Japanese)," (Mika Adachi et al.), Fragrance Journal, Fragrance Journal Ltd., June 15 2005, Vol. 33, No. 6, pages 35-38
[Non-Patent Literature 8] "β-endorphin Stimulates Cytokeratin 16 Expression and Downregulates µ-opiate Receptor Expression in Human Epidermis," (Mei Bigliardi-Qi et al.), The Journal of Investigative Dermatology, USA, Nature Publishing Group, March 2000, Vol. 114, No. 3, pages 527-532
[Non-Patent Literature 9] "µ-opiate Receptor and Beta-endorphin Expression in Nerve Endings and Keratinocytes in Human Skin," (Mei Bigliardi-Qi et al.), Dermatology, Switzerland, Karger, 2004, Vol. 209, No. 3, pages 183-189
[Non-Patent Literature 10] "Experimental and Clinical Pruritus. Studies on Some Putative Peripheral Mediators. The Influence of Ultraviolet Light and Transcutaneous Nerve Stimulation," (B. Fjellner), Acta Dermato-venereologica. Supplementum, Norway, Scandinavian University Press, 1981, Vol. 97, pages 1-34
[Non-Patent Literature 11] "Opioid Peptide Targetting Treatment of Pruritus (in Japanese)," (Kenji Takamori), Rinsho Hifuka (= Clinical Dermatology), Igaku-Shoin Ltd., April 10 2002, Vol. 56, No. 5, pages 145-147
[Non-Patent Literature 12] "Effects of Naltrexone on Spontaneous Itch-associated Responses in NC Mice with Chronic Dermatitis," (Tatsuya Maekawa et al.), Japanese Journal of Pharmacology, The Japanese Pharmacological Society, October 1 2002, Vol. 90, No. 2, pages 193-196
[Non-Patent Literature 13] "Itch-associated Response Induced by Experimental Dry Skin in Mice, " (Takayuki Miyamoto et al.), Japanese Journal of Pharmacology, The Japanese Pharmacological Society, March 1 2002, Vol. 88, No. 3, pages 285-292
[Non-Patent Literature 14] "Imbalance in Opioid System as a Cause of Uremic Pruritus and Effect of a Novel κ-Agonist, TRK-820 (in Japanese)," (Hironari Kumagaya et al.), Sogo Rinsho (= General Clinical Medicine), Nagai Shoten Co., Ltd., May 1 2004, Vol. 53, No. 5, pages 1678-1684
[Non-Patent Literature 15] "Involvement of Central µ-opioid System in the Scratching Behavior in Mice, and the Suppression of It by the Activation of κ-opioid System, " (Hideo Umeuchi et al.), European Journal of Pharmacology, Netherlands, Elsevier Science, 2003, Vol. 447, No. 1, pages 29-35
[Non-Patent Literature 16] "Anti-Pruritic Effect of a Kappa Opioid Receptor Agonist TRK-820," (Hideo Umeuchi et al.), Journal of Pharmacological Sciences, The Japanese Pharmacological Society, March 1 2003, Vol. 91, Supplement No. 1, page 198
[Non-Patent Literature 17] "Knowledge of Diseases Common to Dermatology Necessary for Surgeons 6. Skin Pruritus and Mechanism of Itching (in Japanese)," (Shigeo Kochi), Rinsho Geka (= Clinical Surgery), Igaku-Shoin Ltd., November 20 2001, Vol. 56, No. 12, pages 1522-1524
[Non-Patent Literature 18] B. Wikström et al., J. Am. Soc. Nephrol. 16: 4742-3747 (2005)
[Non-Patent Literature 19] K. Takamori, "Mechanisms and management of intractable pruritus", NII-Electronic Library Service, XP009152671 (2007), pp. 193-197
[Non-Patent Literature 20] H. Kumagai et al., Sogo Rinsho (2004), 53(5), 1678-1684.

### Disclosure of the Invention

### Problems Which the Invention Tries to Solve

The object of this invention is to provide a novel medicine effective for improving skin properties such as preventing skin drying, and/or improving skin roughening against the decline of the skin functions brought about by various causes.

### Means for Solving the Problems

The inventors made an intensive study for solving the abovementioned problems, and as a result, found that a compound having a specific morphinan skeleton or any of its pharmacologically permissible acid addition salts is useful as a skin property-improving therapeutic agent. Thus, this invention has been completed.

This invention relates to a substance according to the following [1] for use in preventing skin drying in patients treated with hemodialysis and/or improving skin roughening in patients treated with hemodialysis.
[1] Substance represented by the following general formula (I): 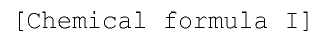 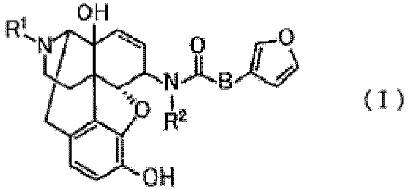 where the double line consisting of a dotted line and a solid line denotes a double bond or single bond; R¹ denotes a cycloalkylalkyl with 4 to 7 carbon atoms; R² denotes a straight chain or branched alkyl with 1 to 5 carbon atoms; and B denotes -CH=CH- or any of its pharmacologically permissible acid addition salts as an active ingredient for use in preventing skin drying in patients treated with hemodialysis and/or improving skin roughening in patients treated with hemodialysiswherein the compound represented by the general formula (I) is (-)-17-(cyclopropylmethyl)-3,14β-dihydroxy-4,5α-epoxy-6β-[N-me-thyl-trans-3-(3-furyl)acrylamido] morphinan.

### Effects of the Invention

This invention provides a skin property-improving therapeutic agent comprising a morphinan derivative or any of its pharmacologically permissible acid addition salts as an active ingredient. Administration of such a skin property-improving therapeutic agent can prevent skin drying, and/or improve skin roughening.

### Best Modes for Carrying out the Invention

The skin property-improving therapeutic agent according to the present invention comprises as an effective component a compound as defined in claim 1.

The dashed line in the Formula (I) represents a single bond.

The skin property-improving according to the present invention comprises as an effective component the compound represented by the already shown Formula (I) as defined above or a pharmaceutically acceptable acid addition salt thereof.

In the formula (I), R¹ denotes cyclopropylmethyl.

R² denotes methyl.

B denotes the trans form of -CH=CH-.

The compound represented by the formula (I) is a compound in which R¹ denotes cyclopropylmethyl; R² denotes methyl; and B denotes trans form -CH=CH-,namely, (-)-17-(cyclopropylmethyl)-3,14β-dihydroxy-4,5α-epoxy-6β-[N-methyl-trans-3-(3-furyl)acrylamido]morphinan.

The compound represented by the Formula (I) may be produced by the method described in Japanese Patent No. 2525552, Japanese Patent No. 2525552, in Chem. Pharm. Bull., 52, 664(2004) and Japanese Patent No. 2525552; Bioorg. Med. Chem. Lett., 5, 1505(1995)) as a raw material and in Chem. Pharm. Bull., 52, 664(2004).

Further, the skin property-improving therapeutic agent of this invention is used as a skin drying preventive and/or a skin roughening preventive in patients treated with hemodialysis. It is desirable that the skin property-improving therapeutic agent is used as oral medicine, but it is not limited to such formulations, being able to be used as skin medicine for external application, unless the effects are impaired.

The pharmacologically permissible acid addition salts of this invention include inorganic acid salts such as hydrochlorides, sulfates, nitrates, hydrobromides, hydroiodides and phosphates, organic carboxylates such as acetates, lactates, citrates, oxalates, glutarates, malates, tartrates, fumarates, mandelates, maleates, benzoates and phthalates, organic sulfonates such as methanesulfonates, ethanesulfonates, benzenesulfonates, p-toluenesulfonates and camphorsulfonates. Above all, hydrochlorides, hydrobromides, phosphates, tartrates, methanesulfonates can be preferably used.

Further, the compound represented by the formula (I) or any of its pharmacologically permissible acid addition salts is purified for medicinal application and subjected to a necessary safety test, and the compound that has passed the test can be orally administered as it is or as a medicinal composition obtained by mixing the compound with a publicly known pharmacologically permissible acid, carrier or excipient. The formulation for oral administration can be selected from tablets, capsules, powder, granules, though not of course limited to them. These formulations can be prepared by well-known methods usually used in the area of medicines. The dosage of the skin property-improving therapeutic agent of this invention can be adequately set based on the symptom, the age and weight of the patient, administration method. Usually the amount of the active ingredient per adult per day is about 0.1 µg to about 100 mg in case of oral administration and about 0.01 µg to about 10 mg in case of non-oral administration.

Any of the skin property-improving therapeutic agent of this invention can be administered alone and can also be administered together with another drug, for example, a moisture retaining agent, anti-itching agent for external application or steroid ointment. Examples of the moisture retaining agent include vaseline, urea, heparinoid ointment, *Artemisia princes*-mixed ointment, ceramide-containing cream and *Camellia japonica*-oil lotion. Examples of the anti-itching agent for external application include antihistamine ointment and crotamiton ointment. In the case where itching is strong, a steroid ointment can also be further used together. The skin property-improving therapeutic agents of this invention can be used as moisture retaining agents, skin barrier function restoration promoters, skin drying preventives and skin roughening preventives.

### [Example]

This invention is explained below in more detail in reference to an example.

### Example 1

2.5 µg of (-)-17-(cyclopropylmethyl)-3,14β-dihydroxy-4, 5α-epoxy-6β-[N-methyl-trans-3-(3-furyl)acrylamido]-morphinan hydrochloride (compound 1) represented by the following structural formula (II) was hermetically contained in soft capsules formed of a gelatin film, to prepare an oral medicine. Two capsules (5.0 µg) of the oral medicine were administered to each of two itch patients treated with hemodialysis and having resistance against the conventional treatment. After start of administration, one patient (female, age 79) began to feel less itching and became smoother on the skin in general (especially legs), to have beautiful skin. The other patient (male, age 69) who had been very poor in complexion and skin texture became less dry on the face and gained good complexion after start of administration with the oral medicine. About 4 to 5 days after start of administration with the oral medicine, when one of the patients was relieved of the itching felt on the portions ranging from the wrists to the shoulders and on the breasts, the patient began to have moist skin, but after end of administration, when the patient began to feel itching again, the patient began to have dry skin.

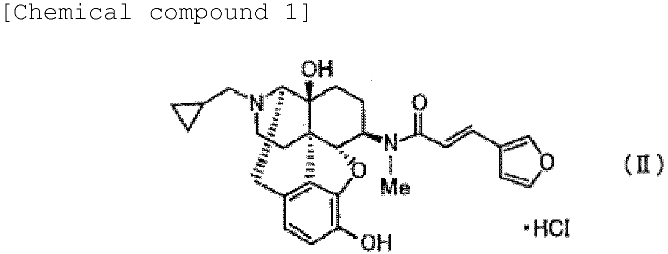

### Industrial Applicability

This invention is useful as a skin property-improving therapeutic agent that can improve skin roughening, and/or prevent skin drying in patients treated with hemodialysis and give a moisture retaining effect.

## Claims

1. A substance represented by the following formula (I): 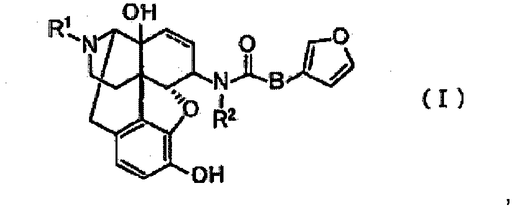 where the double line consisting of a dotted line and a solid line denotes a double bond or single bond; R¹ denotes a cycloalkylalkyl with 4 to 7 carbon atoms; R² denotes a straight chain or branched alkyl with 1 to 5 carbon atoms; and B denotes -CH=CH- or any of its pharmacologically permissible acid addition salts, for use in preventing skin drying in patients treated with hemodialysis and/or improving skin roughening in patients treated with hemodialysis,
wherein the compound represented by the formula (I) is (-)-17-(cyclopropylmethyl)-3,14β-dihydroxy-4,5α-epoxy-6β-[N-methyl-trans-3-(3-furyl)acrylamido]morphinan.

## Patentansprüche

1. Substanz, repräsentiert durch die folgende Formel (I): 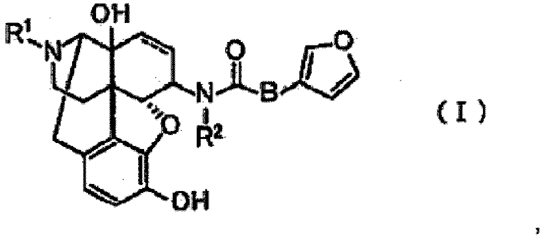 wobei die Doppellinie, die aus einer gestrichelten Linie und einer durchgezogenen Linie besteht, eine Doppelbindung oder Einfachbindung bezeichnet; R¹ ein Cycloalkylalkyl bezeichnet, das 4 bis 7 Kohlenstoffatomen aufweist; R² ein geradkettiges oder verzweigtes Alkyl bezeichnet, das 1 bis 5 Kohlenstoffatome aufweist; und B -CH=CH- oder irgendeines seiner pharmakologisch zulässigen Säureadditionssalze bezeichnet, zur Verwendung beim Vorbeugen von Hautaustrocknung in mit Hämodialyse behandelten Patienten und/oder zum Verbessern der Hautrauheit in mit Hämodialyse behandelten Patienten,
wobei die Verbindung, die durch die Formel (I) repräsentiert ist, (-)-17-(Cyclopropylmethyl)-3,14β-dihydroxy-4,5α-epoxy-6β[N-methyl-trans-3-(3-furyl)-acrylamid]morphinan ist.

## Revendications

1. Substance représentée par la formule (I) suivante : 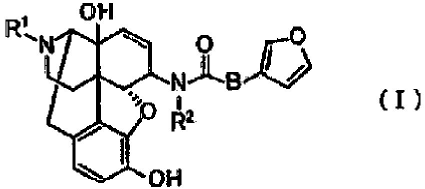 où le double trait constitué d'un trait pointillé et d'un trait continu désigne une double liaison ou une simple liaison ; R¹ désigne un cycloalkylalkyle avec 4 à 7 atomes de carbone ; R² désigne un alkyle à chaîne linéaire ou ramifié avec 1 à 5 atomes de carbone ; et B désigne -CH=CH- ou l'un quelconque de ses sels d'addition d'acide pharmacologiquement acceptables, pour son utilisation dans la prévention de la sécheresse cutanée chez des patients traités par hémodialyse et/ou l'amélioration de la rugosité de la peau chez les patients traités par hémodialyse,
dans laquelle le composé représenté par la formule (I) est le (-)-17-(cyclopropylméthyl)-3,14β-dihydroxy-4,5α-époxy-6β-[N-méthyl-trans-3-(3-furyl)acrylamido]morphinane.
